# EUROPEAN PATENT SPECIFICATION

(11) **EP 2 684 483 B1**
(45) Date of publication and mention of the grant of the patent: **14.12.2016**
(21) Application number: 12176319.7
(22) Date of filing: 13.07.2012
(51) Int. Cl.: A45D 26/00

(54) **Epilation device with an improved functional head**
Epilationsgerät mit einem verbesserten Funktionskopf
Dispositif d'épilation avec une tête fonctionnelle améliorée

(43) Date of publication of application: 15.01.2014
(73) Proprietor: Braun GmbH, 61476 Kronberg (DE)
(72) Inventor: Pfeiffer, Bernd, 65824 Schwalbach (DE); Lippold, Frank, 64287 Darmstadt (DE); Heilig, Bernhard, 74743 Seckach (DE)
(74) Representative: Schneider, Stefan Michael

(56) References cited:
- EP-A1- 2 127 552
- EP-A2- 0 513 900
- DE-A1-102004 047 873
- JP-A- 1 172 502
- US-A1- 2011 005 079

## Description

### FIELD OF THE INVENTION

The present invention relates to a functional head for an epilation device according to the preamble of claim 1

### BACKGROUND OF THE INVENTION

In many areas of mechanical engineering, there are endeavours to mechanically optimise and reduce friction of moving parts, particularly drive parts or also functional heads of a tool which operates with moving parts. These efforts also relate to mechanical devices for personal beauty care, such as razors or epilation devices. Epilation devices are mechanical devices which can pluck out hairs with the aid of tweezer elements, to remove undesired body hairs

EP 513900 B1 discloses such an epilation device. This device has a drive unit and a functional head (epilation head). The epilation head may have various types of configurations. A suitable configuration includes an arrangement of discs which rotate about a longitudinal axis and may move together and apart in the axial direction. This movement may bring about a clamping contact and a plucking of the body hair. In such an arrangement, it is quite evident that frictional forces can occur, particularly between non-moving parts of the epilation head and moving parts which move against the non-moving parts.

German patent application DE 102004047873 A1 discloses an epilation head for an epilation device, having a different configuration. The device has a housing which serves as a handpiece, and which accommodates a motor drive. An epilation head is snugly attached to the housing, which epilation head has a rotating cylinder. The rotating cylinder is rotatably mounted in the epilation head, so as to rotate around a shaft. This rotating cylinder has a plurality of clamp elements which can engage hairs and are well suited for the function of the device. The clamp elements in this instance are not disc-shaped but tweezers-shaped. The cylinder has a plurality of individual clamp elements which can be actuated by actuating elements which extend parallelly to the axis of rotation. Each clamp element is associated with such an actuating element, so that a plurality of actuating elements can be operated. The actuating elements essentially are resemble tappets, having a head which comes into contact with the given clamp element, and a foot whereby the actuation of a clamp element is initiated. For this initiation, application elements are provided which act on the actuating elements. According to this disclosure, the essential parts of the device, even the parts of the rotating cylinder, are preferably fabricated from plastic. In order to reduce the friction between the control surfaces of the application elements and the actuating elements, a supply means which dispenses a lubricant is provided. Lubrication occurs when the control surfaces have lubricant applied to them. A channel can also serve to store the lubricant. As lubricant, a solid lubricant is preferred.

The usage habits of epilation devices have been undergoing change. Increasingly, the devices are used in a wet environment, for example in a shower. This imposes new requirements on the design of the devices. With such a mode of use, no longer are the devices subjected to only occasional exposure to water and even surfactants (for example, when being cleaned), but now the devices must endure such exposure over a relatively long period of use. The presence of water and/or surfactants makes it difficult to avoid at least some washing-out of the lubricant. Accordingly, an underlying problem of the present invention was to provide a device wherein the necessary lubrication is ensured over a long period of time, ideally the longest expected service life of the device, and is also ensured under conditions where in each use the device may come into contact with water and surfactants

The present invention provides a solution to this problem, and a means of avoiding the known drawbacks of the state of the art. In this way, it is intended to provide a device which is inexpensive to manufacture and is reliable in operation.

### SUMMARY OF THE INVENTION

The present invention relates to a functional head according to claim 1 which can be generally employed in a device for industrial or private use, particularly in a device for beauty care, such as an epilation device. More particularly, the invention relates to a functional head having at least one first element which has a frictional surface, and at least one second element which is movable over the frictional surface when mechanical friction is overcome, wherein a lubricating device is provided by which a lubricant may be applied to the frictional surface, wherein the lubricant is accommodated in a lubricant reservoir and the lubricant is transported to the frictional surface by means of at least one outlet opening, and wherein an increase of mechanical friction by the movement of the at least one second element over the frictional surface leads to heating of at least parts of the lubricant reservoir.

The actual configuration of the functional head may vary widely, depending on the particular device, but will always involve two elements which are movable with respect to each other. In the context of the invention, the functional head may perform the main function of the device, and as a rule the functional head is mounted exteriorly on the device and is accessible. However, it is also possible for the functional head to perform an internal function in the device, wherewith it will not be accessible, or visible, from the exterior.

According to the present invention it is provided that the functional head has at least one first element which has a frictional surface. This frictional surface may have any convenient configuration; frequently the frictional surface will be a flat running surface, in individual cases the running surface will be path-like or circular-path-like. Multiple first elements may also be provided, for example two, three, or four first elements, each of which has a frictional surface. For example, with an epilation device it is possible that two first elements are symmetrically disposed, and are disposed on opposite ends of a shaft or axis. Moreover, according to the present invention, at least one second element is provided, which is movable over the frictional surface, under overcoming of mechanical friction. Often, the second element will be pressed against the frictional surface, so that an enhanced mechanical friction occurs, enhanced by the pressure. Ordinarily, the mechanical friction will be such that lubrication would seem necessary in order to provide for use over a long period. The exact nature of the movement over the frictional surface is not essential -- frequently it may consist of movement over a closed path, for example a circular path. In the context of the invention, multiple second elements may be provided, for example two, three, or four second elements. The second elements may also be symmetrically disposed, for example at opposite ends of an axis or a shaft.

Moreover, a lubrication device is provided on the functional head. The lubrication device will be able to dispense a lubricant which may be applied to the frictional surface. For this purpose, a lubricant reservoir is provided, which accommodates the lubricant. The lubricant may be transported to the frictional surface via at least one outlet opening. The outlet opening will be adjusted to the particular type of lubricant. In the case of a relatively fluid lubricant of low viscosity, a very small outlet opening is advantageous, for example in the form of an orifice.

The increased mechanical friction by the movement of the at least one second element over the frictional surface (in general on the at least one first element) leads to heating of at least parts of the lubricant reservoir. It is well known that friction can lead to heating, so that the exact embodiment is not essential for the invention. Conceivably, there are means for intentionally increasing the friction in the case of inadequate lubrication. This higher friction may lead to greater heating. This heating has effects on at least parts of the lubricant reservoir. In this way, the lubricant stored there is heated. If a solid lubricant is used, the heating may liquefy it. A highly viscous lubricant may be rendered less viscous by the heating. The liquefaction or general reduction of the viscosity of the lubricant facilitates the exit of the lubricant through the outlet opening. The transition from a solid lubricant to a liquid lubricant may allow the lubricant to pass through the outlet opening, whilst prior to that event such passage would not have occurred.

Thus, the present invention allows the lubrication to be adjusted to the need for lubrication. For this purpose, the phenomenon of heating is drawn upon. In this way, an increased amount of lubricant may be dispensed in response to a situation of deficient lubrication. In this way, a relatively small lubricant reservoir may be employed to maintain lubrication of the device over a very long time.

The present invention is particularly advantageous for functional heads which are made from plastic parts. Plastic parts are particularly susceptible to wear. On the other hand, plastic parts are often fabricated in a solid and heavy configuration, which promotes heat transfer through such parts.

The functional head has a rotating cylinder and is to be used in an epilation device,. The rotating cylinder is be equipped with at least one clamp device for plucking of hairs. To actuate such a clamp device, at least one rod (for example a tappet-like rod) may be provided. Typically, exactly one such rod is provided for each clamp device. The rod may has a foot and a head. Here, the head of the rod may act on the clamp device. The foot of the rod may serve as a second element, in the context of the present invention. Thus, the foot of the rod is movable over the frictional surface, with overcoming of mechanical friction.

Advantageous is a functional head, particularly for an epilation device, which has at least one non-movable side piece. A non-movable side piece facilitates the installation of the functional head in an epilation device. The movement of parts of the functional head, particularly a rotating cylinder, relative to the non-movable side piece, may also be utilized for control of the functional head, thus for example for control of the clamp elements. Advantageously, a functional head is provided wherein a non-movable side piece has a frictional surface and wherein the first element is in the form of such a non-movable side piece. In such a functional head, thus the tappet-like rods move over the frictional surface with overcoming of mechanical friction, which frictional surface is disposed on the non-movable side piece. Advantageously the functional head has two side pieces disposed oppositely from each other. The frictional surface of the non-movable side piece may advantageously be provided as an application surface for the at least one tappet-like rod. If one tappet-like rod is provided per each clamp element, a plurality of tappet-like rods will be passing over the frictional surface. Typically, the foot of each tappet-like rod will rest against the frictional surface. It is advantageous if a plurality of feet of rods are arranged to form a circumferential path which runs over the frictional surface. This allows one to fabricate a very compact functional head. With this design, unavoidably, high frictional resistances occur, which may be reduced however by the lubrication device according to the present invention. Advantageously with such a functional head, control elements are provided on the frictional surface, which elements may act on the foot of the tappet-like rod. Such control elements are as a rule in the form of raised areas. Advantageously the control elements comprise nub surfaces.

The lubrication device may advantageously be provided at a rotating element or a static element of the functional head. Both solutions have certain advantages and disadvantages. Both solutions may be made more effective by a specific configuration.

If the lubrication device is provided on a rotating element, that element may be the second element. For example, advantageously the lubrication device may be provided on the rotating cylinder of a functional head for an epilation device. If the lubrication device is provided on a rotating element, the at least one outlet opening may be disposed such that the lubricant is transported to the frictional surface with the aid of centrifugal forces. For example, lubricant may be liquefied by the heating of at least parts of the lubricant reservoir. To the extent that the lubricant is disposed behind the outlet opening, the centrifugal forces may cause lubricant to pass through the outlet opening.

The lubrication device may also be provided on a static element. This static element may be the first element, which has a frictional surface. For example, the lubrication device may be provided on the non-movable side piece of the functional head. Because the friction between the first and second element depends only on the relative movement, a static element may also be heated by friction. It is advantageous if the outlet opening is disposed close to a movable element, such that an air stream which arises with the movement of the movable element promotes the transport [of the lubricant] to the frictional surface. As a rule, the movement of a movable element relative to the static element will be accompanied by a certain air stream. In a particular case, this air stream may be undesirable, but it is almost impossible to avoid. In the context of the present invention, such an air stream may be employed advantageously for transport of lubricant, for example in the form of particles or droplets. In this manner, it is possible to supply lubricant to the frictional surface without utilising centrifugal forces. If present, the outlet openings should be disposed such that they are upstream of the frictional surface, with regard to the air stream. If a sufficient air stream is generated, the lubrication reservoir can also be an open system. Particularly, if a lubricant of low viscosity is used, such as paraffin, no cover or cover plate (and hence no outlet openings) are required for the lubrication reservoir. It is also conceivable to influence the guiding of the air stream by suitable means, in order to optimally take advantage of the air stream for lubrication purposes.

An advantageous lubricant reservoir may be provided in a hollow space in a component part of a functional head. This hollow space may then be closed off by a fixedly or removably mounted cover plate. Outlet openings may be provided in this cover plate. The lubricant may be stored in a porous foam material. The porous foam material may be connected to the cover plate. In this way, the lubricant reservoir may be conveniently filled during the manufacturing process.

### BRIEF DESCRIPTION OF THE DRAWINGS

- FIG.1: is a plan view of an epilation device which may be provided with a functional head according to the present invention, in the form of an epilation head;
- FIG. 2: is a perspective view of a functional head according to the present invention, in the form of a functional head for an epilation device, wherein the view is exploded as to certain parts;
- FIG. 3: is an enlarged perspective view of details of the functional head from Fig. 2; and
- FIG. 4: is a perspective view of a lubrication reservoir according to the present invention, together with surrounding parts.

### DETAILED DESCRIPTION OF THE INVENTION

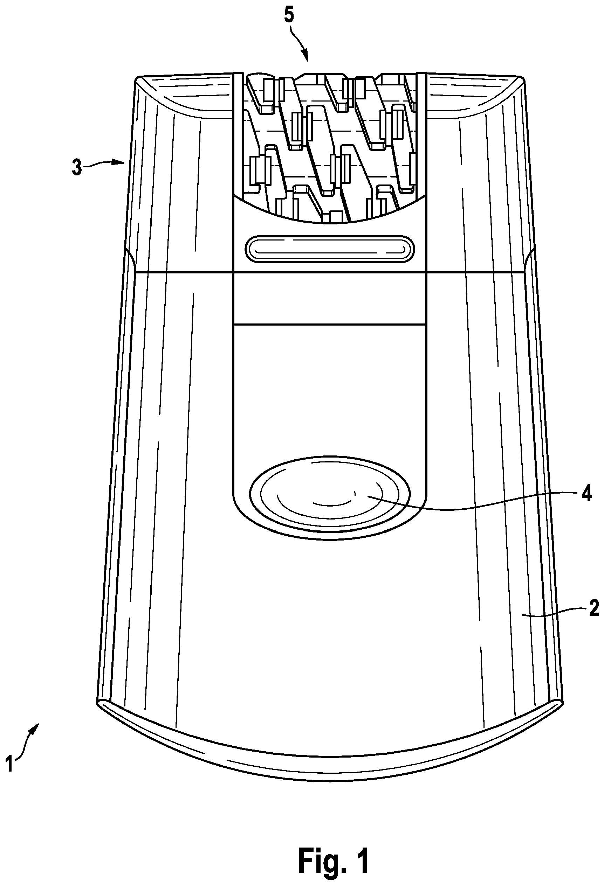

FIG. 1 illustrates an epilation device 1 which may be fitted with a functional head according to the present invention. The epilation device is shown in a slightly simplified schematic lateral view. The epilation device 1 has a handpiece 2. The handpiece 2 has a drive, accommodated in housing. The drive (not shown here) includes as a rule an electric motor and drive elements.

Also, a rechargeable battery may be integrated into the handpiece 2. With the use of a handpiece with a rechargeable battery, it is critical that the functional head operate easily. Accordingly, the lubrication according to the present invention is a significant help. In particular, for reasons of safety, if the epilation device is to be used in a shower or bathtub it should be battery-operated (rechargeable or otherwise). Commonly, a person who buys such a device expects to use it for an extended period of time in the wet state. As already mentioned, use of the device in a wet state, particularly for an extended period, can lead to insufficient lubrication. This problem is solved by the lubrication device according to the present invention.

The epilation head 3 is mounted on the handpiece 2. Another main component of the device is the switch 4 disposed in the centre of the front side of the housing. The switch is employed to start in motion the rotating cylinder of the epilation head 3, to carry out epilation. The working region 5 of the epilation head, in which the rotating cylinder operates, may be seen. One may see on the surface of the rotating cylinder a plurality of plucking units, each of which has a closable plucking gap. This plucking gap is created by two clamping elements which may be moved toward one another and in this ways may accomplish the plucking movement. It is possible to combine the handpiece 2 with another functional head, in certain units. Such a functional head may have a hair removal unit in the form of a razor or trimmer. It is also conceivable to have a functional head with a massage function. The lubricating device according to the present invention is also suitable for such alternative functional heads with mechanical drives.

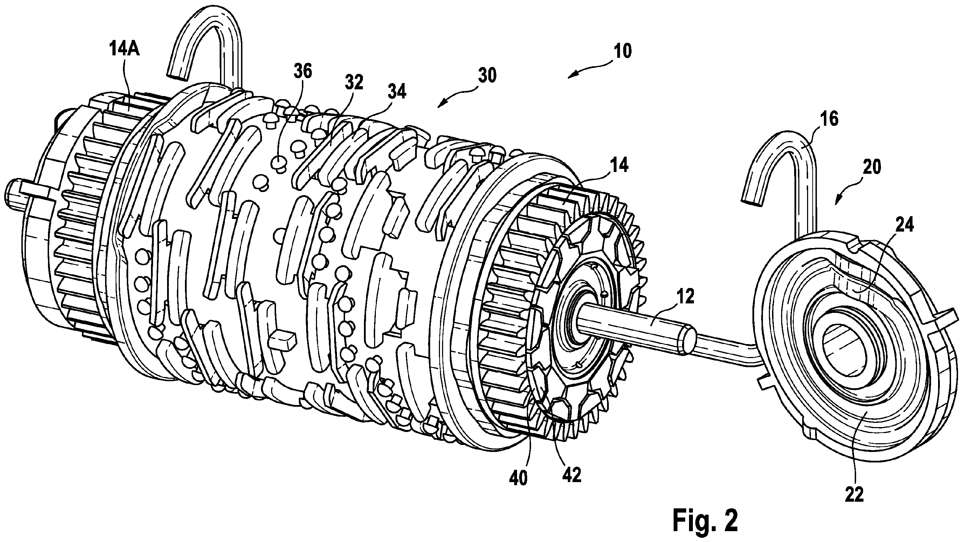

Fig. 2 is a perspective view of a rotating cylinder 10 such as may be used as a main component in an epilation head. This rotating cylinder is shown in a non-mounted partial exploded view. The rotating cylinder 10 may rotate around a shaft 12. The drive means are provided by the gear 14 and the corresponding gear 14A. These gears may be engaged by corresponding gears of a drive. The rotating cylinder 10 may be connected with other elements with the aid of the clip 16. In particular, the rotating cylinder 10 may be firmly connected to a side piece 20 by the clip 16 (the clip 16 and side piece 20 are not shown in the mounted position but in a partially exploded view). Ordinarily, side pieces 20 will be provided on both sides of the rotating cylinder 10, which side pieces will come to be applied over the shaft 12, so that the two side pieces 20 will be disposed so that they face each other in mirror image symmetry, and enclose the rotating cylinder 10 between them.

The side piece 20 has an application surface 22 for fixing and controlling the rotating cylinder, which surface 22 is oriented toward the rotating cylinder, wherein a nub-bearing surface is disposed on this surface.

The rotating cylinder 10 has a functioning surface 30. Movable clamp pieces 32 and fixed clamp pieces 34 are positioned on this functioning surface. The fixed clamp pieces may be fabricated from plastic. This alternative is appropriate in particular when other main components of the rotating cylinder are also fabricated from plastic. The movable clamp pieces may also be fabricated from plastic; however, it may often be advantageous to fabricate them from metal. Also, hair guide elements 36 are provided on the functioning surface 30 of the rotating cylinder 10. These may assist in improving the passage of hairs between the plucking gaps disposed between the clamp pieces.

Tappet-like rods 40 are provided, for mechanical actuation of the movable clamp pieces 32. Because of the plurality of movable clamp pieces 32 on the functioning surface 30, as a rule also a plurality of tappet-like rods 40 will be required. As a rule, one tappet-like rod 40 is provided for each movable clamp piece 32. Therefore, the tappet-like rods 40 must be densely arranged. In this perspective view, one sees essentially the foot 42 of the tappet-like rod; the closely arranged feet of the rods form an encircling path on the side of the rotating cylinder 10.

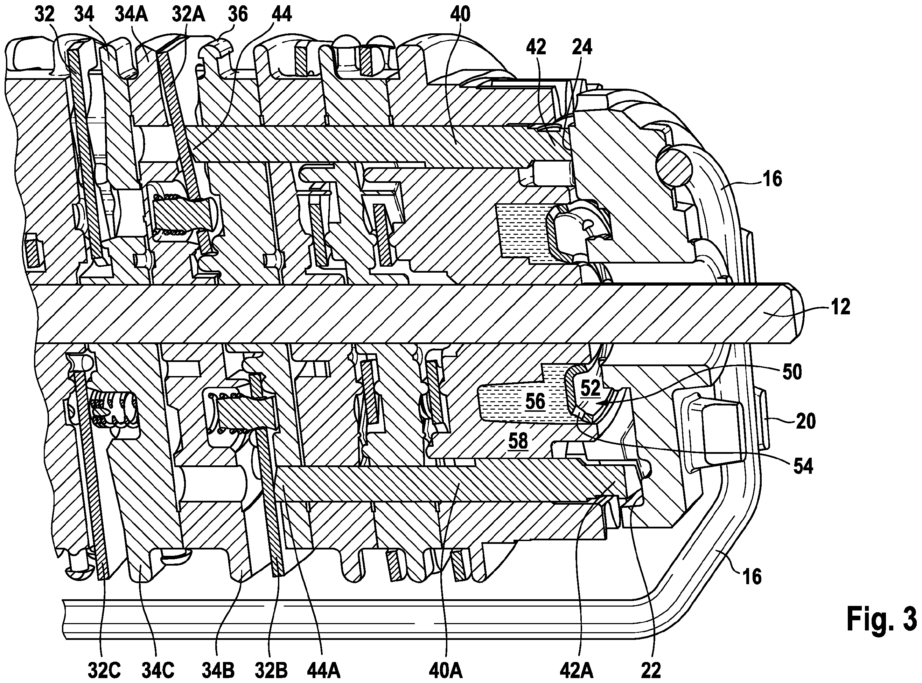

The lubricating device according to the present invention is disposed between the shaft 12 of the rotating cylinder 10 and the tappet-like rods 40. This device 50 is thus disposed around the shaft 12 in a path which is closer to the shaft than the tappet-like rods (the arrangement is evident from Fig. 3).

FIG. 3 is a partial perspective view of the rotating cylinder according to FIG. 2. Here, no part of the view is exploded; i.e., all elements are in their working positions. Also, the view is enlarged with respect to FIG. 2, and the rotating cylinder and side piece are shown in a partial cross section through the shaft 12.

The rotating cylinder is shown with its essential component parts. In particular, one can see the movable clamp pieces 32 (in particular, the movable clamp pieces with the reference numerals 32A, 32B, and 32C) and the corresponding fixed clamp pieces 34 (in particular, the fixed clamp pieces with the reference numerals 34A, 34B, and 34C). Tappet-like rods 40 are guided in the rotating cylinder body; these rods have a foot 42 directed outward and a head 44 directed inward. A plurality of tappet-like rods is provided, which extend essentially parallelly to the shaft 12 and are disposed in radially opposed positions. Thus, the tappet-like rod 40 is disposed oppositely to a tappet-like rod 40A, which latter has a foot 42A and a head 44A. Each such tappet-like rod operates an associated clamp element. This occurs by pressing of the tappet-like rod inward toward the centre of the rotating cylinder. This pressure must correspondingly act first on the foot 42 of the tappet-like rod.

The side piece 20 serves to control the clamp elements via the tappet-like rods 40. This side piece 20 is pressed against the sides of the rotating cylinder by the clip 16. Accordingly, the side piece 20 has an application surface 22 on its inner side. Even if this [surface 22] is not in constant pressing contact with the tappet-like rods 40, the rods will nonetheless be held in the cylinder by the application surface 22 of the side piece 20. The nub surface 24 of the side piece 20 may exert pressure on the feet of the tappet-like rods. This can be seen above the shaft for the tappet-like rod foot 42. It is in direct contact with the nub surface 24 of the side piece 20. In contrast, the foot 42A of the tappet-like rod is not in contact with the application surface, in the position illustrated. As a result, no pressure is exerted on the movable clamping element 32B. As a result, there is a gap between the clamping element 32B and the corresponding fixed clamping element 34B, as may be seen in the Figure. In contrast, the clamping element 32 is in contact with the clamping element 34, so that a hair may be engaged in this plucking gap and may be held by the pinching action.

As may be seen from this functional description, the control of the tappet-like rods 40 by the side piece 20 is important for the operation of the functional head. As a result of the either constant or very frequently repeated contact between the tappet-like rod feet and a surface of the side piece, which functions as a frictional surface, for example the nub surface 24, an unavoidable friction occurs.

To reduce this friction, according to the present invention a lubricating device 50 is provided. This may be seen well in the cross section of FIG. 3. It includes a cover plate 52. This cover plate has orifices 54 in it. The cover plate covers a reservoir 56 which has lubricant in it. This reservoir 56 is disposed in a hollow space of a component part of the rotating cylinder, and is delimited by wall elements 58.

If, as a result of insufficient lubricant, a high friction develops between the feet 42 of the tappet-like rods and the side piece 20, the side piece and the feet 42 of the tappet-like rods will heat up. This will give rise to heating of the neighbourhood, which will cause the lubricant reservoir 56 to heat up. Moreover, heat conduction will occur, wherein the external parts of the rotating cylinder will be heated by the tappet-like rods or the increased temperature of the direct neighbourhood. In particular, the walls 58 of the lubricant reservoir 56 will be heated. The heating reduces the viscosity of the lubricant, which lubricant may be, for example, solid or semisolid, wherewith the heating will at least partially liquefy the lubricant. Consequently, particles of lubricant will pass through the outlet openings 54 on the cover plate 52.

For many functional heads, it may be useful for heat to be conducted also over the cover plate. The cover plate is disposed close to the surfaces where the friction is occurring. Particularly if the choice has been made to fabricate the cover plate from metal, the heat transport may be rapid.

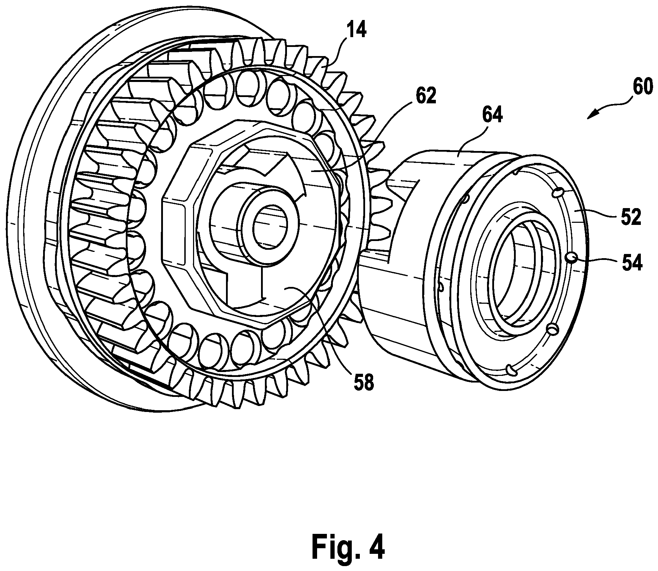

FIG. 4 shows a perspective view of a lubricating device 50 according to the present invention, the device comprising, as an essential element, cartridge 60. Such a cartridge can be placed in the receiving cavity 62. This receiving cavity in general is positioned in the area of the functional head. In the depicted embodiment the receiving space is positioned in a portion of the gear 14, which has been shown in Fig. 2. The wall elements 58 of the part, which comprises gear 14, also serve as walls of the lubricating device 50. In this embodiment, however, a storage element 64 is provided. The storage element 64 can store a lubricant, for example paraffin. Useful is an open-pored foam, such as a highly porous metal or plastic foam. The element 64 can be directly joined to the closure 52, for example by adhesive joining. The closure 52 is shown with the known orifices 54. By using storage element 64 a cartridge forming a separate element can be created. this element can be prepared in a separate production process and then be placed in the designated receiving cavity 62 of the functional head. In this manner the production of the lubricating device can be made independent of the production of the functional head. Further it can be avoided to inject liquid or semiliquid paraffin into the receiving cavity of a functional head directly. Such paraffin can be received by the storage element 64 and the element will then be joined as one unit, suitably together with the closure 52 into the functional head.

The description and associated drawings show the manner, in which a functional head with good lubrication and therefore long service life may be fabricated.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A functional head for an epilation device (1) having a rotating cylinder (10) with at least one clamping device for plucking of hairs, wherein at least one rod (40) is provided for actuating the clamping device, and the rod (40) has a foot (42) and a head (44), wherein the head of the rod (40) acts on the clamping device, having at least one first element which has a frictional surface, and at least one second element which is movable over the frictional surface when mechanical friction is overcome, wherein the second element is provided in the form of the foot (42) of the rod (40), and wherein a lubricating device (50) is provided by which a lubricant may be applied to the frictional surface, wherein the lubricant is housed in a lubricant reservoir (56), and the lubricant is transported to the frictional surface by means of at least one outlet opening (54), wherein the lubricant is a solid or highly viscous lubricant and wherein an increase of mechanical friction by the movement of the at least one foot (42) of the rod (40) over the frictional surface leads to heating of at least parts of the lubricant reservoir (56) and lubricant stored there such that solid lubricant liquefies or highly viscous lubricant reduces viscosity, wherein the transition to the liquefied or lower viscosity lubricant allows the lubricant to pass through the outlet opening (54).

2. The functional head according to claim 1, wherein the rod (40) is a tappet-like rod.

3. The functional head according to claim 1 or 2, having a least one non-movable side piece 20 which has a frictional surface, and wherein the first element is provided in the form of the non-moving side piece.

4. The functional head according to claim 2, which has at least one non-movable side piece 20 which has a frictional surface, and wherein the first element is provided in the form of the non-movable side piece 20, and wherein the frictional surface is provided as an application surface 22 for the at least one tappet-like rod 40.

5. The functional head according to one of claims 2 to 4, wherein control elements are provided on the frictional surface, which control elements 24 may act on the foot 42 of the tappet-like rod 40.

6. The functional head according to one of the preceding claims, wherein the lubricating device 50 is provided on a rotating element.

7. The functional head according to the preceding claim, wherein the at least one outlet opening 54 is designed such that the lubricant is transported to the frictional surface with the aid of centrifugal forces.

8. The functional head according to claim 6 or 7, wherein the lubricant reservoir 56 is covered by a cover plate 52 which has at least one outlet opening 54 on a peripheral circle around the rotational axis of the rotating element.

9. The functional head according to one of claims 1 to 5, wherein the lubricating device 50 is provided on a static element.

10. The functional head according to the preceding claim, wherein the outlet opening 54 is disposed close to a movable element, such that an air stream which arises on the occasion of movement of the movable element promotes the transport of lubricant to the frictional surface.

## Patentansprüche

1. Funktionskopf für ein Epilationsgerät (1) mit einem Drehzylinder (10) mit wenigstens einer Klemmeinrichtung zum Auszupfen von Haaren, wobei mindestens eine Stange (40) zum Betätigen der Klemmeinrichtung bereitgestellt ist, und die Stange (40) einen Fuß (42) und ein Kopfstück (44) hat,
wobei das Kopfstück der Stange (40) auf die Klemmeinrichtung wirkt und wenigstens ein erstes Element mit einer Reibungsfläche und wenigstens ein zweites Element, das über der Reibungsfläche bewegbar ist, wenn mechanische Reibung überwunden wird, wobei das zweite Element in Form des Fußes (42) der Stange (40) bereitgestellt ist, und wobei eine Schmiereinrichtung (50) bereitgestellt ist, durch die ein Schmiermittel auf der Reibungsfläche aufgebracht werden kann, wobei das Schmiermittel in einem Schmiermittel-Vorratsbehälter (56) untergebracht ist, und
wobei das Schmiermittel mittels wenigstens einer Auslassöffnung (54) auf der Reibungsfläche aufgebracht wird, wobei das Schmiermittel ein festes oder hochviskoses Schmiermittel ist, und wobei eine Erhöhung der mechanischen Reibung durch die Bewegung durch den mindestens einen Fuß (42) der Stange (40) über die Reibungsfläche zum Erwärmen mindestens von Teilen des Schmiermittel-Vorratsbehälters (56) und des dort gespeicherten Schmiermittels führt, sodass sich festes Schmiermittel verflüssigt oder hochviskoses Schmiermittel die Viskosität reduziert, wobei es der Übergang zum verflüssigten Schmiermittel oder zum Schmiermittel geringerer Viskosität dem Schmiermittel ermöglicht, durch die Auslassöffnung (54) zu gelangen.

2. Funktionskopf nach Anspruch 1, wobei die Stange (40) eine stößelähnliche Stange ist.

3. Funktionskopf nach Anspruch 1 oder 2, der mindestens ein nicht bewegliches Seitenstück 20 hat, das eine Reibungsfläche hat, und wobei das erste Element in Form des nicht beweglichen Seitenstücks bereitgestellt ist.

4. Funktionskopf nach Anspruch 2, der mindestens ein nicht bewegliches Seitenstück 20 hat, das eine Reibungsfläche hat, und wobei das erste Element in Form des nicht beweglichen Seitenstücks 20 bereitgestellt ist, und wobei die Reibungsfläche als Anbringungsfläche 22 für die mindestens eine stößelähnliche Stange 40 bereitgestellt ist.

5. Funktionskopf nach einem der Ansprüche 2 bis 4, wobei Steuerelemente auf der Reibungsfläche bereitgestellt sind, und wobei diese Steuerelemente 24 auf den Fuß 42 der stößelähnlichen Stange 40 einwirken können.

6. Funktionskopf nach einem der vorstehenden Ansprüche, wobei die Schmiereinrichtung 50 an einem rotierenden Element bereitgestellt ist.

7. Funktionskopf nach dem vorstehenden Anspruch, wobei die mindestens eine Auslassöffnung 54 so konzipiert ist, dass das Schmiermittel mithilfe von Zentrifugalkräften zur Reibungsfläche transportiert wird.

8. Funktionskopf nach Anspruch 6 oder 7, wobei der Schmiermittel-Vorratsbehälter 56 durch eine Abdeckplatte 52 bedeckt ist, die mindestens eine Auslassöffnung 54 auf einem Umfangskreis um die Drehachse des rotierenden Elements hat.

9. Funktionskopf nach einem der Ansprüche 1 bis 5, wobei die Schmiereinrichtung 50 an einem statischen Element bereitgestellt ist.

10. Funktionskopf nach dem vorstehenden Anspruch, wobei die Auslassöffnung 54 nahe an einem beweglichen Element angeordnet ist, sodass ein Luftstrom, der in Zusammenhang mit der Bewegung des beweglichen Elements aufkommt, den Transport von Schmiermittel zur Reibungsfläche fördert.

## Revendications

1. Tête fonctionnelle destinée à un dispositif d'épilation (1) possédant un cylindre rotatif (10) avec au moins un dispositif de serrage pour l'épilation de poils, dans laquelle au moins une tige (40) est fournie pour actionner le dispositif de serrage, et la tige (40) possède un pied (42) et une tête (44),
dans laquelle la tête de la tige (40) agit sur le dispositif de serrage, possédant au moins un premier élément qui a une surface de frottement, et au moins un deuxième élément qui est mobile sur la surface de frottement lorsque le frottement mécanique est surmonté, dans laquelle le deuxième élément se présente sous la forme du pied (42) de la tige (40), et dans laquelle un dispositif lubrifiant (50) est fourni par lequel un lubrifiant peut être appliqué à la surface de frottement, dans laquelle le lubrifiant est logé dans un réservoir de lubrifiant (56), et
le lubrifiant est transporté vers la surface de frottement au moyen d'au moins une ouverture de sortie (54), dans laquelle le lubrifiant est un lubrifiant solide ou très visqueux et dans laquelle une augmentation de frottement mécanique par le mouvement du au moins un pied (42) de la tige (40) sur la surface de frottement entraîne un échauffement d'au moins des parties du réservoir de lubrifiant (56) et du lubrifiant qui y est stocké de telle sorte que le lubrifiant solide se liquéfie ou que le lubrifiant très visqueux diminue de viscosité, dans laquelle le passage au lubrifiant liquéfié ou de plus basse viscosité permet au lubrifiant de traverser l'ouverture de sortie (54).

2. Tête fonctionnelle selon la revendication 1, dans laquelle la tige (40) est une tige de type poussoir.

3. Tête fonctionnelle selon la revendication 1 ou 2, possédant au moins une pièce latérale non mobile (20) qui a une surface de frottement, et dans laquelle le premier élément se présente sous la forme de la pièce latérale non mobile.

4. Tête fonctionnelle selon la revendication 2, qui possède au moins une pièce latérale non mobile (20) qui a une surface de frottement, et dans laquelle le premier élément se présente sous la forme de la pièce latérale non mobile (20), et dans laquelle la surface de frottement est fournie en tant que surface d'application (22) pour l'au moins une tige de type poussoir (40).

5. Tête fonctionnelle selon l'une des revendications 2 à 4, dans laquelle des éléments de commande sont fournis sur la surface de frottement, lesquels élément de commande (24) peuvent agir sur le pied (42) de la tige de type poussoir (40).

6. Tête fonctionnelle selon l'une des revendications précédentes, dans laquelle le dispositif lubrifiant (50) est fourni sur un élément rotatif.

7. Tête fonctionnelle selon la revendication précédente, dans laquelle ladite au moins une ouverture de sortie (54) est conçue de telle sorte que le lubrifiant est transporté vers la surface de frottement avec l'aide de forces centrifuges.

8. Tête fonctionnelle selon la revendication 6 ou 7, dans laquelle le réservoir de lubrifiant (56) est couvert par une plaque de couverture (52) qui comporte au moins une ouverture de sortie (54) sur un cercle périphérique autour de l'axe de rotation de l'élément rotatif.

9. Tête fonctionnelle selon l'une des revendications 1 à 5, dans laquelle le dispositif lubrifiant (50) est fourni sur un élément statique.

10. Tête fonctionnelle selon la revendication précédente, dans laquelle l'ouverture de sortie (54) est disposée à proximité d'un élément mobile, de telle sorte qu'un courant d'air qui survient à l'occasion d'un mouvement de l'élément mobile favorise le transport de lubrifiant vers la surface de frottement.
